(19) Europäisches Patentamt
European Patent Office
Office européen des brevets

(11) EP 3 888 623 A1

(12) EUROPEAN PATENT APPLICATION

(43) Date of publication:
06.10.2021 Bulletin 2021/40

(51) Int Cl.:
A61K 8/02 (2006.01)     A61K 8/19 (2006.01)
A61K 8/24 (2006.01)     A61Q 11/00 (2006.01)

(21) Application number: 20167833.1

(22) Date of filing: 02.04.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Unilever Global IP Ltd
Wirral, CH62 4ZD (GB)

(72) Inventors:
• ASHCROFT, Alexander Thomas.
  Mollington
  Cheshire
  CH1 6LD (GB)
• LIMER, Adam John.
  Bebington, Wirral
  Merseyside
  CH63 3JW (GB)
• ZHANG, Meili
  Taringa
  Queensland 4068 (AU)

(74) Representative: Tansley, Sally Elizabeth
Unilever PLC
Unilever Patent Group
Bronland 14
6708 WH Wageningen (NL)

(54) **ORAL CARE SYSTEM**

(57)     A system for mitigating the sensitivity of teeth the system comprising a first anhydrous composition comprising:
i) particulate calcium carbonate;
ii) a water insoluble and/or slightly soluble calcium source;
a second aqueous composition comprising a phosphate source.

EP 3 888 623 A1

**Description**

**Field of the Invention**

The present invention relates to a system for treating tooth hypersensitivity.

**Background**

Tooth hypersensitivity is a common but painful condition affecting up to 20% of the adult population. Hypersensitive teeth may be sensitive to cold, heat, air or sugary foods.

The incidence of tooth hypersensitivity increases with age. Tooth hypersensitivity is believed to be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed. Dentinal tubules are naturally present in the dentinal layer of the tooth and they provide for an osmotic flow between the inner pulp region of the tooth and the outer root surfaces.

The currently accepted theory for tooth hypersensitivity is the hydrodynamic theory, based on the belief that open exposed dentinal tubules allow fluid flow through the tubules. This flow excites the nerve endings in the dental pulp. Clinical replicas of sensitive teeth viewed in a scanning electron microscope (SEM) reveal varying numbers of open or partially occluded dentinal tubules.

There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

An example of an occluding agent is found in US 5,270,031 which describes a tubule occluding desensitizer comprising a polyacrylic acid such as Carbopol® polymeric materials. Another tubule occluding composition is disclosed in US 5,374,417 which discloses a potassium salt of a synthetic anionic polymer, such as a polycarboxylate.

WO2014/023466, WO2014/023465 and WO2015/067422 disclose compositions for treating tooth hypersensitivity comprising calcium carbonate.

There is a continuing need for providing effective systems for delivering desensitizing agents to the teeth which can provide treatment and relief of hypersensitivity.

**Description of the Invention**

The present invention relates a system for mitigating the sensitivity of teeth the system comprising: a first anhydrous composition comprising:

   i) particulate calcium carbonate;
   ii) a water insoluble and/or slightly soluble calcium source;
   a second aqueous composition comprising a phosphate source.

**Detailed Description of the Invention**

The first composition comprises calcium carbonate, preferably particulate calcium carbonate composed of primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

In one present invention, a suitable source of particulate calcium carbonate as defined above includes crystalline calcium carbonates in which the individual crystals have a prismatic morphology and an average size of 2 microns or less.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline forms of calcium carbonate are available naturally, or may be synthetically produced in three particular crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.

References to a "prismatic crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are prismatic crystals. Prismatic crystals have a set of faces with parallel edges that are parallel to the vertical or "c"-axis direction. There can be three, four, six, eight or even twelve faces that can form a prism. For the purposes of the present invention, preferred prismatic crystals have a generally uniform, typically generally hexagonal cross-sectional shape.

References to a "scalenohedral crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are scalenohedral crystals. Scalenohedral crystals have a set of faces that are inclined to the vertical or "c"-axis direction, each of which are scalene triangles (i.e. triangles whose three sides are unequal in length). For the purposes of the present invention, preferred scalenohedral crystals have a trigonal scalenohedral shape, with twelve scalene triangle faces.

Reference to acicular" in this context refers to the shape of the crystals. Usually, crystals grow in three directions, length, width and height. Some crystals however, have one or two preferred growth directions. Acicular crystals have a preferred crystal growth in one direction. Examples are crystals in the form of needles, rods, fibres, whiskers or columns and the like. For the purposes of the present invention, preferred acicular crystals are rod-like or needle-like with a generally uniform, typically generally circular, cross-sectional shape.

Crystal shape may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particles and aggregates or agglomerates.

Usually, crystals grow in three directions, length, width and height. Some crystals however, have one or two preferred growth directions. For the purposes of the present invention, preferred prismatic calcium carbonate crystals have a ratio between the length and the width of the crystal (the so-called aspect ratio) ranging from about 1:1 up to about 3:1 (length:width). The aspect ratio of a particle (e.g. a crystal) may typically be obtained from SEM photographs by averaging the ratio between the length and width of the particle, measured on at least 10 particles for 1 photograph.

Preferably the calcium carbonate for use in this invention has a prismatic, scalenohedral or acicular crystal morphology.

For the purposes of the present invention, preferred prismatic calcium carbonate crystals have an average size which is less than 2 microns and generally ranges from about 0.1 to 2 microns, with a preferred size ranging from about 0.2 to about 1.5, more preferably from about 0.3 to about 1, most preferably from about 0.5 to 0.9 microns. Particle (e.g. crystal) size may be determined by standard techniques known to those skilled in the art such as sedimentation. Particle size values obtained from sedimentation techniques are normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population.

For the purposes of the present invention, preferred scalenohedral calcium carbonate crystals have an average size which is less than 2 microns and generally ranges from about 0.05 to about 1.5 microns, with a preferred size ranging from about 0.1 to about 1 micron, more preferably from about 0.15 to about 0.5 micron, most preferably from about 0.2 to 0.35 micron. A standard technique known to those skilled in the art for determining the average size of the individual crystals is air permeametry according to the Lea and Nurse method (standards NF X 11-601 and NF X 11602). This analytical technique determines the average particle size by measuring the pressure drop across a packed powder bed using a water manometer. The pressure drop is a function of the permeability of the packed bed. This is related to the surface area of the particles which is then transformed to an average size based upon the assumption that the particles are spherical. Average particle size (Dp) is obtained from the massic area (SM) derived from the Lea and Nurse method by making the assumptions that all the particles are spherical, non-porous and of equal diameter, and by neglecting contact surfaces between the particles. The relationship between Dp and SM is the following:

$$Dp=6/(\rho SM)$$

where $\rho$ is the specific mass of the calcium carbonate. The average particle size (Dp) obtained in this way represents the average equivalent spherical diameter, or average ESD, where equivalent spherical diameter (ESD) is defined as the diameter of a notional sphere having the same volume as the particle.

For the purposes of the invention preferred acicular calcium carbonate is composed of a length of 2 microns or greater. Preferred acicular calcium carbonate crystals for use in the invention have a length ranging from 2 to 100 microns, more preferably from 10 to 30 microns and a width ranging from 0.1 to 4.0 microns, more preferably from 0.5 to 1.0 microns. A specific class of material suitable for use in the invention includes aragonite crystal form calcium carbonates such as those described for example in US 5,164,172.

The ratio between the length and the width of a crystal, the so-called aspect ratio, is higher than 1:1 for preferred acicular crystals. The higher the aspect ratio, the longer the crystal. For the purposes of the present invention the aspect ratio is preferably at least 2.5:1, more preferably at least 10:1. For example the aspect ratio may typically range from 2.5:1 to 200:1, and preferably ranges from 10:1 to 60:1, more preferably from 20:1 to 30:1.

In a preferred embodiment to calcium carbonate has a particulate size less than 2 microns.

Preferred calcium carbonate crystals, in particular scalenohedral prismatic calcium carbonate generally have a BET specific surface area higher than or equal to 0.1 m2/g, preferably higher than or equal to 1 m2/g, more preferably higher than or equal to 3 m2/g and most preferably higher than or equal to 4 m2/g. The calcium carbonate particles according to the invention generally have a BET specific surface area lower than or equal to 30 m2/g, preferably lower than or equal to 25 m2/g, more preferably lower than or equal to 20 m2/g, and most preferably lower than or equal to 15 m2/g. The BET specific surface area is usually measured according to the standard ISO 9277 which specifies the determination of the overall specific external and internal surface area of disperse or porous solids by measuring the amount of physically adsorbed gas according to the Brunauer, Emmett and Teller (BET) method.

Crystalline forms of calcium carbonate and suitable for use in the invention are available naturally, or may be produced by precipitation production technology. Typically, precipitated calcium carbonate is prepared by exposing calcium hydroxide slurry to a carbonation reaction. Control of the specific solution environment during the nucleation and growth of calcium carbonate governs the size and the shape of the crystals in the resulting precipitated calcium carbonate product.

A commercially available source of particulate prismatic calcium carbonate suitable for use in the invention is ViCALity® ALBAFIL® Precipitated Calcium Carbonate (PCC), ex Specialty Minerals Inc., Bethlehem, Pa. 18017.

A commercially available source of particulate scalenohedral calcium carbonate suitable for use in the invention is SOCAL® S2E Precipitated Calcium Carbonate (PCC), ex Solvay S.A.

Suitable acicular crystal form calcium carbonates for use in the invention are commercially available and include those marketed by Maruo Calcium Company Limited, Japan under the trade name WISCAL®.

Mixtures of any of the above described materials may also be used.

The level of the first composition is preferably from 0.5 to 15 wt% of the total first composition, preferably from 1 to 10 wt%.

The first composition comprises a calcium source that is insoluble or slightly soluble in water.

Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. In a preferred embodiment, the calcium source is calcium silicate. In a more preferred embodiment, the calcium silicate used is ($CaSiO_3$) whereby the same is made commercially available under the name Microcal ET by Ineos Silicas, Ltd.

In yet another preferred embodiment, the calcium source is insoluble calcium silicate, present as the composite material calcium oxide-silica ($CaO$-$SiO_2$).

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often-preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the first composition is typically from 0.1 to 50%, and preferably, from 1 to 30%, and most preferably, from 5 to 20% by weight of the oral care composition based on total weight of the oral care composition and including all ranges subsumed therein.

A preferred form of calcium silicate is calcium silicate coated $TiO_2$. Examples of preferred forms of calcium silicate coated TO02 are disclosed in WO2012/031786 and WO2012/031785.

The first composition comprises an anhydrous base formulation in that the composition comprises less than 1wt% of the activator of the total composition of water; preferably less than 0.5 wt%, more preferably less than 0.1 wt% of the total first composition.

The second composition comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 1 to 12%, and most preferably, from 2 to 9% by weight of the composition, based on total weight of the composition in which it is included and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The first or second phase of the oral care composition described may independent of each other comprise ingredients which are common in the art, such as:

■ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);

■ anti-inflammatory agents such as ibuprofen, flurbiprofen, aspirin, indomethacin, etc.;

■ anti-caries agents such as sodium trimetaphosphate and casein;

■ plaque buffers such as urea, calcium lactate, calcium glycerophosphate and polyacrylates;

■ vitamins such as Vitamins A, C and E;

■ plant extracts;

■ desensitizing agents, e.g. potassium citrate, potassium chloride, potassium tartrate, potassium bicarbonate, potassium oxalate, and potassium nitrate;

■ anti-calculus agents, e.g. alkali-metal pyrophosphates, hypophosphite-containing polymers, organic phosphonates and phosphocitrates, etc;

■ biomolecules, e.g. bacteriocins, antibodies, enzymes, etc ;

■ flavors, e.g., peppermint and spearmint oils;

■ proteinaceous materials such as collagen;

■ preservatives;

■ opacifying agents;

■ coloring agents like FD&C blue, yellow and/or red dyes/colorants;

■ pH-adjusting agents;

■ sweetening agents;

■ surfactants, such as anionic, cationic and zwitterionic or amphoteric surfactants (e.g., sodium lauryl sulfate, sodium dodecylbenzene sulfonate);

■ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;

■ fluoride sources like sodium fluoride, stannous fluoride, sodium monofluorophosphate, zinc ammonium fluoride, tin ammonium fluoride, calcium fluoride, cobalt ammonium fluoride or mixtures thereof;

■ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and

■ other optional ingredients that may be included are, e.g., bleaching agents such as peroxy compound, e.g., potassium peroxydiphosphate, effervescing systems such as sodium bicarbonate/citric acid systems, color change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care system of the present invention, in particular the activation system and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

Preferably both compositions of the invention have a viscosity from 10,000 to 60,000 cps at 20°C. More preferably, the viscosity is from 15,000 to 45,000 cps at 25°C, more preferably from 19,000 to 35,000 cps at 20°C.

In the context of the present invention viscosity is measured at 20°C, using a Brookfield viscometer with Helipath Stand and a T-bar, TB spindle, rotating at 5 rpm. The measurement jar has a minimum diameter of 5 cm and a minimum height of 10 cm. The viscosity value is recorded 60 seconds from the start of the measurement.

Mode of Use

The invention provides a method of de-sensitizing teeth. The composition first composition is mixed with the second composition preferably at a weight ratio of first composition to second composition from 1:5 to 5:1 prior to application to the teeth and is applied to the teeth using a finger or an applicator.

Preferably the application of the oral care product of the invention is carried out once daily for a period of several consecutive days, in addition to a regular regime of tooth brushing (preferably at least twice daily).

The invention will now be illustrated by the following non-limiting Examples:

## Examples

### Preparing Simulated Oral Fluid:

A simulated oral fluid was prepared by adding 1.9L of water to a glass beaker. The following materials were added one by one with continuous stirring, allowing sufficient time for each chemical to fully dissolve before adding the next. Sodium chloride 16.07g, sodium hydrogen carbonate 0.7g, potassium chloride 0.448g, potassium hydrogen phosphate 2.56g, magnesium chloride hexahydrate 0.622g, 1M hydrochloric acid 40ml, calcium chloride 0.1998g and sodium sulfate 0.1434g. The pH was adjusted to 7.0 using saturated TRIS buffer and the total volume made up to 2L using a volumetric flask.

**Scoring Standard for Tubules Blockage:**

Regardless of the original shape of the dentine disks, a square (with a size of 4mm × 4mm) is selected and one image is captured under 50x magnification. Within this square, five spots (each with a size of 150 micro etaeta × 150 micro etaeta, one in the middle, and one in every comer) are selected and observed under 1000x magnification. The blockage of tubules are accessed following the standards described below. The measurement is carried out for the two dentine disks of each test group.

| Score | Tubules Blockage |
|---|---|
| 0 | All dentinal tubules are open |
| 1 | <20% of dentinal tubules are fully blocked |
| 2 | 20 to 50% of dentinal tubules are fully blocked |
| 3 | 50 to 80% of dentinal tubules are fully blocked |
| 4 | 80 to 100% of dentinal tubules are fully blocked |
| 5 | All dentinal tubules are fully blocked. |

**Dentine disc preparation:**

Human dentine discs with thickness of 2.0 mm were prepared by cutting them from extracted human teeth. The dentine discs were polished on a series of wet silicon-carbide papers ranging from 280 to 600 grit, so that to standardise the tested surfaces as the same condition. The dentin surfaces were then treated by 37% phosphoric acid for 60 seconds to remove the smear layer created during the grinding process and to open and enlarge all its dentin tubules.

**Example 1**

A first formulation was prepared dispersing 0.1 g of xanthan gum until it dissolved at 80°C in 79.9g of glycerol. To this mixture was added 15g of calcium silicate and 5g of Omyacare HP900 fine ground natural chalk.
A second formulation was prepared by combining 2g sodium carboxymethyl cellulose, 0.1g sodium saccharine, 0.64g sodium fluoride, 3.2g sodium phosphate, 3.8g trisodium phosphate, 0.3g benzyl alcohol, 0.3g phenoxyethanol and 0.3g ethylhexyl glycerine in 89.36g water. The materials were mixed until a homogeneous solution was obtained.
Four human dentine disks were separated into two groups (n=2).
2g of first formulation and 2g of the second formulation were mixed, the dentine discs were soaked in the resulting slurry for 15 minutes at 37°C. After the samples were removed from the slurry, rinsed in 40 ml water and mixed gently for 1 minute. Samples were then incubated in simulated oral fluid for 6 hours. The dentine disks were brushed with de-ionised water using a tooth brushing machine equipped with toothbrushes. The load of the tooth brushing was 175 g +1-5 g, with a brushing speed of 150 rpm for 1 minute. This cycle was completed 5 times in total.
The tubule blocking efficacy was assessed after 3 and 5 applications.

| | 3 Applications | 5 Applications |
|---|---|---|
| Example 1 | 3.1 ±0.15 | 5 |

Example A:

The ingredients of the dentifrice formulations are shown in the table below. The pastes were prepared by mixing (in weight percent) the following ingredients under moderate shear until a homogeneous composition was obtained.

| Ingredient | Level wt% |
|---|---|
| Fine Ground Natural Chalk | 38 |
| OmyaCare HP900 | 2 |
| Sorbitol (70% Solution) | 20 |
| Water | 28.61 |

(continued)

| Ingredient | Level wt% |
| --- | --- |
| Hydrated Silica | 3.5 |
| Sodium lauryl sulfate | 2.5 |
| Sodium Silicate (30% solution) | 1.5 |
| Sodium monofluorophosphate | 1.11 |
| Sodium carboxymethyl cellulose | 0.63 |
| Flavour | 0.9 |
| Titanium Dioxide | 0.5 |
| Benzyl Alcohol | 0.5 |
| Sodium Saccharine | 0.25 |

To evaluate blockage efficacy of dentinal tubules, the test samples was mixed with water at a ratio of 4 g to 8 mL water to make a slurry.

Two dentine disks were brushed with the slurry under a tooth brushing machine equipped with toothbrushes. The load of the tooth brushing was 175 g and the automatic brushing operated at a speed of 150 rpm. After brushing for 1 min, the dentine disks were soaked in the toothpaste slurry for 1 min. Then the dentine disks were rinsed with distilled water, 40ml, and placed in simulated oral fluid (SOF) under the condition of a shaking water bath at 37 degrees centigrade at 60.0 rpm. The disks were soaked for a total of 4 hours. After this time the disks were removed from the SOF and the application and soaking protocol repeated. The brushing was repeated three times on the first day, then the dentine disks were kept in SOF overnight (>12 hours) in a shaking water bath at 37 degrees centigrade to mimic oral environment. A further two applications were made on the second day with a total of five product applications.

The tubule blocking efficacy was assessed after 3 and 5 applications.

| | 3 Applications | 5 Applications |
| --- | --- | --- |
| Example A | 2.6 ±0.22 | 3.1 ±0.48 |

The results in example 1A and 1B show significantly greater dentine occlusion than example A.

**Claims**

1. A system for mitigating the sensitivity of teeth the system comprising a first anhydrous composition comprising,

    i) particulate calcium carbonate;
    ii) a water insoluble and/or slightly soluble calcium source;
    a second aqueous composition comprising a phosphate source.

2. A composition according to any preceding claim in which the calcium carbonate comprises primary particles which are acicular and which have a length of 2 microns or greater.

3. A composition according to any preceding claim in which the calcium carbonate has a average particle size of less than 2 microns.

4. A composition according to claim 1 in which the calcium carbonate comprise of scalenohedral calcium carbonate crystals have an average size which is less than 2 microns.

5. A composition according to any one of claim 1 in which the calcium carbonate comprise of prismatic calcium carbonate crystals have an average size which is less than 2 microns.

6. A composition according to any preceding claim in which the first composition comprises a phosphate source.

7.  A system according to any preceding in which the phosphate source comprises trisodium phosphate, sodium dihydrogen phosphate or mixtures thereof.

8.  A system according to any preceding claim in which the phosphate source is also present in the first composition.

9.  A composition according to any preceding in which the calcium source is calcium silicate.

10. A composition according to any preceding claim which further comprises a polysaccharide.

11. A composition according to any preceding claim which further comprises xanthan gum.

12. A composition according to any preceding claim in which the first composition has has a viscosity at 20°C from 15,000 to 45,000 cps.

13. A composition according to any preceding claim in which the second composition has a viscosity at 20°C from 15,000 to 45,000 cps.

Europäisches
Patentamt

European
Patent Office

Office européen
des brevets

# EUROPEAN SEARCH REPORT

Application Number

EP 20 16 7833

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/067422 A1 (UNILEVER PLC [GB]; UNILEVER NV [NL]; CONOPCO INC DBA UNILEVER [US]) 14 May 2015 (2015-05-14) * claims 1-12; example all * | 1-13 | INV. A61K8/02 A61K8/19 A61K8/24 A61Q11/00 |
| X | WO 2014/023465 A1 (UNILEVER PLC [GB]; UNILEVER NV [NL]; CONOPCO INC [US]) 13 February 2014 (2014-02-13) * pages 15-19; table 1 * | 1-13 | |
| X | WO 2014/023466 A1 (UNILEVER PLC [GB]; UNILEVER NV [NL]; CONOPCO INC DBA UNILEVER [US]) 13 February 2014 (2014-02-13) * pages 14-17; table 1 * | 1-13 | |
| X | WO 2018/108389 A1 (UNILEVER NV [NL]; UNILEVER PLC [GB]; CONOPCO INC D/B/A UNILEVER [US]) 21 June 2018 (2018-06-21) * claim all; example all * | 1-13 | |
| A | WO 2013/041419 A1 (UNILEVER PLC [GB]; UNILEVER NV [NL] ET AL.) 28 March 2013 (2013-03-28) * examples 1-2 * | 1-13 | TECHNICAL FIELDS SEARCHED (IPC) A61K A61Q |
| A | WO 2016/192925 A1 (UNILEVER PLC [GB]; UNILEVER NV [NL]; CONOPCO INC D/B/A UNILEVER [US]) 8 December 2016 (2016-12-08) * page 9, line 11 - page 10, line 3; claims 1-10 * | 1-13 | |

The present search report has been drawn up for all claims

| Place of search | Date of completion of the search | Examiner |
|---|---|---|
| Munich | 26 June 2020 | Nopper, Agathe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 7833

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2020

| Patent document cited in search report | | Publication date | Patent family member(s) | | Publication date |
|---|---|---|---|---|---|
| WO 2015067422 | A1 | 14-05-2015 | BR 112016007010 A2<br>CL 2016001019 A1<br>CN 105682747 A<br>EP 3065833 A1<br>WO 2015067422 A1 | | 01-08-2017<br>24-02-2017<br>15-06-2016<br>14-09-2016<br>14-05-2015 |
| WO 2014023465 | A1 | 13-02-2014 | BR 112015002551 A2<br>CL 2015000283 A1<br>CN 104684619 A<br>EA 201590140 A1<br>EP 2882500 A1<br>PH 12015500240 A1<br>WO 2014023465 A1 | | 04-07-2017<br>02-10-2015<br>03-06-2015<br>30-07-2015<br>17-06-2015<br>30-03-2015<br>13-02-2014 |
| WO 2014023466 | A1 | 13-02-2014 | BR 112015002531 A2<br>CL 2015000284 A1<br>CN 104684618 A<br>EA 201590141 A1<br>EP 2882499 A1<br>PH 12015500239 A1<br>WO 2014023466 A1 | | 04-07-2017<br>28-08-2015<br>03-06-2015<br>30-07-2015<br>17-06-2015<br>30-03-2015<br>13-02-2014 |
| WO 2018108389 | A1 | 21-06-2018 | BR 112019010091 A2<br>CL 2019001624 A1<br>CN 110062617 A<br>EP 3554459 A1<br>PH 12019501105 A1<br>WO 2018108389 A1 | | 01-10-2019<br>27-12-2019<br>26-07-2019<br>23-10-2019<br>20-01-2020<br>21-06-2018 |
| WO 2013041419 | A1 | 28-03-2013 | BR 112014006689 A2<br>CL 2014000697 A1<br>CN 103930089 A<br>EP 2758026 A1<br>US 2015050322 A1<br>WO 2013041419 A1 | | 04-04-2017<br>09-01-2015<br>16-07-2014<br>30-07-2014<br>19-02-2015<br>28-03-2013 |
| WO 2016192925 | A1 | 08-12-2016 | BR 112017023500 A2<br>CL 2017003084 A1<br>CN 107690327 A<br>EA 201792166 A1<br>EP 3302350 A1<br>PH 12017502039 A1<br>US 2018140520 A1<br>WO 2016192925 A1 | | 31-07-2018<br>11-05-2018<br>13-02-2018<br>31-05-2018<br>11-04-2018<br>23-04-2018<br>24-05-2018<br>08-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5270031 A
- US 5374417 A
- WO 2014023466 A
- WO 2014023465 A
- WO 2015067422 A
- US 5164172 A
- WO 2008015117 A
- WO 2012031786 A
- WO 2012031785 A
- DE 03942643 A